# EUROPEAN PATENT SPECIFICATION

(11) **EP 2 948 432 B1**
(45) Date of publication and mention of the grant of the patent: **19.09.2018**
(21) Application number: 13702763.7
(22) Date of filing: 24.01.2013
(51) Int. Cl.: C07D 223/16

(54) **PROCESS FOR MAKING IVABRADINE**
VERFAHREN ZUR HERSTELLUNG VON IVABRADIN
PROCÉDÉ DE PRÉPARATION D'IVABRADINE

(43) Date of publication of application: 02.12.2015
(73) Proprietor: Synthon BV, 6545 CM Nijmegen (NL)
(72) Inventor: MELSA, Petr, 67817 Blansko (CZ); ZABADAL, Miroslav, 67817 Blansko (CZ)
(74) Representative: Mendivil Gil, Maria Dolores
(86) International application number: PCT/EP2013/051370
(87) International publication number: WO 2014/114341

(56) References cited:
- WO-A1-2011/138625
- WO-A1-2013/024400
- WO-A2-2008/146308

## Description

Ivabradine, chemically 3-[3-({[(7*S*)-3,4-dimethoxybicyclo[4.2.0]octa-1,3,5-trien-7-yl]methyl}(methyl)amino)propyl]-7,8-dimethoxy-2,3,4,5-tetrahydro-1H-3-benzazepin-2-one of formula (I) 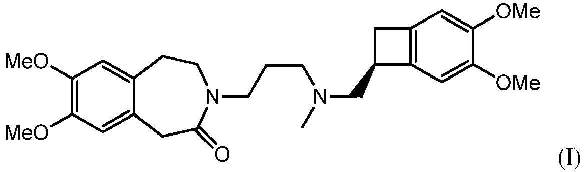 is a pharmaceutically active substance, which is used for the treatment of stable angina pectoris. It reduces the heart rate by a mechanism different from beta-blockers and calcium channel blockers, which are commonly prescribed anti-anginal drugs.

Compound (I) has one chiral carbon atom in position 7. Ivabradine is the (S)-enantiomer.

In the marketed product, which are film-coated tablets for oral administration and are sold, e.g., under the trade name Procorolan by Servier, ivabradine hydrochloride is the active pharmaceutical ingredient.

Ivabradine hydrochloride was first disclosed in EP 534859 (US 5296482).

Various crystalline forms (i.e. polymorphs) of ivabradine hydrochloride, each of which is characterized by a certain distinct X-Ray Powder Diffraction (XRPD) pattern, are known in the art.

Alpha- crystalline form has been disclosed in US 7176197. This form is an anhydrous form, which takes up water under prolonged standing upon transformation into the hydrated form beta.

Beta- crystalline form has been disclosed in US 2006/0194962. This form is a hydrate (most probably a tetrahydrate) and, if heated, it is converted to an anhydrous beta-d crystalline form as shown in US 2006/0194965.

Gamma- crystalline form has been disclosed in US 2006/0194963. This form is a hydrate and, if heated, it is converted to an anhydrous gamma-d crystalline form as shown in US 2006/0194964.

EP 1775288 (US 2007/0082885) disclosed the so called delta-form of crystalline ivabradine hydrochloride. The crystalline form delta is a non-stoichiometric hydrate comprising about 2.8% of water. Internal analysis showed that also the product obtained by the process disclosed in EP 534859 is the form delta.

EP 1775287 (US 2007/0082886) disclosed the so called delta-d form of crystalline ivabradine hydrochloride. It is an anhydrous product.

Other salts of ivabradine were disclosed in WO2009/124940 (hydrobromide), WO 2008/146308 (oxalate) WO 2011/138626 (nitrate, perchlorate, hydroiodide), WO 2011/104723 (tartrate, citrate, hydrogensulfate), WO 2011/157720 (adipate, mandelate, aspartate, malate, esylate, hydroxynaphthoate).

Ivabradine (and acid addition salts thereof) can be produced by various known processes.

One of such known processes has been disclosed in EP 534859 and is based on catalytic hydrogenation of the compound of formula (II), which will be further denoted hereinbelow as dehydroivabradine.

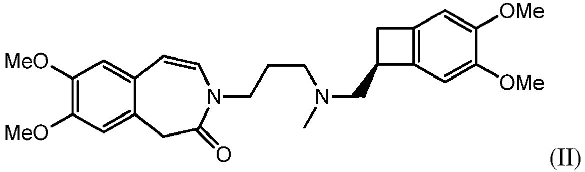

The dehydroivabradine of formula (II) is made by condensation of the compounds of formula (III) and (IV)

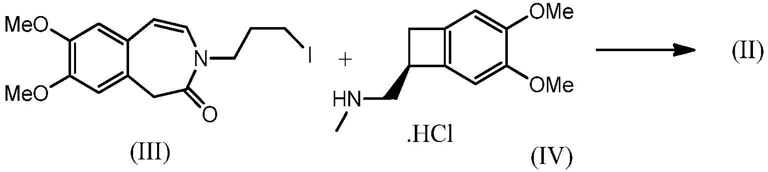

In the published example of said process, the condensation between (III) and racemic (IV) proceeded by 18 hours reflux in acetone in the presence of potassium carbonate. The reaction mixture was evaporated, dissolved in ethyl acetate and extracted by aqueous HCl. After neutralization of the acidic aqueous phase, the product was extracted back to ethyl acetate. The extract was dried and evaporated under formation of an oil, which was chromatographed on a column and the corresponding fraction was evaporated. Yield of racemic dehydroivabradine was 64%. The product was hydrogenated in glacial acetic acid by 4.9 bar pressure of hydrogen in the presence of palladium hydroxide catalyst at ambient temperature for 24 hours. After removal of the catalyst, the mixture was evaporated and partitioned between water and ethyl acetate. After neutralization of the aqueous phase, the product was extracted into ethyl acetate. The organic phase was dried and evaporated and the residue was purified by column chromatography. The corresponding fraction was evaporated and the residue was recrystallized from ethyl acetate yielding racemic ivabradine in 40% yield.

The same process was used for making ivabradine starting from optically active compound (IV), i.e. (S)-enantiomer. Yields were not reported, but one may expect very similar yields were obtained.

It is immediately apparent that the prior art process exhibits several drawbacks, such as long reaction times (18 and 24 hours, resp.) at high temperature (reflux), low yields (64 and 40%, resp.), and the requirement of chromatographic purification.

WO 2011/138625 improved the prior art process by reacting compound (IV) with the chloride of formula (IIIa) 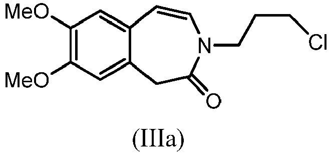 in the presence of sodium iodide and a base (thus, it may be expected that the iodo-compound of formula (III) is temporarily formed), wherein the formed dehydroivabradine of formula (II) is isolated from the reaction mixture in the form of an acid addition salt, preferably as an oxalate or nitrate, and is purified by recrystallization. The isolated salt of (II) is then converted to the free base, and the base is hydrogenated in alcohol or acetic acid. Ivabradine is isolated from the reaction medium as an acid addition salt.

In a typical arrangement shown in the examples of WO 2011/138625, the solvent for the reaction of (IIIa) with (IV) was 1-methyl-2-pyrrolidone, the amount of sodium iodide used was 2-5 molar equivalents, the reaction time was 6-18 hours, and the temperature was 40-80°C (typically, 8.5 hours at 60°C). The reaction mixture was partitioned between water and ethyl acetate, the organic phase was extracted with aqueous HCl, and the combined aqueous phases were neutralized and extracted with ethyl acetate. The combined organic phases were concentrated by evaporation, and the concentrate was treated with a solution of oxalic acid in methanol. Crystals of the oxalate salt of (II) were separated and isolated in a yield of 76%.

The free base of the compound (II) was liberated from the oxalate salt by potassium carbonate and by extraction from aqueous medium to ethyl acetate. The ethyl acetate solution was evaporated and the residuum was hydrogenated in methanol-HCl mixture over Pd-C catalyst at 1-15 bar at 25-100°C, typically at 10 bar and 45°C for about 6 hours. The reaction mixture was evaporated and the residuum treated with acetonitrile. Ivabradine hydrochloride precipitated therefrom and was isolated in 77.5 % yield.

The need of isolation of the intermediate as a salt and the relatively complicated treatment of such salt before hydrogenation is a disadvantage of this prior art process.

It is apparent that several ways to make ivabradine exist, but that each of them is associated with certain drawbacks. Thus, there exists an objective need for an improvement in the process for making ivabradine and the hydrochloride salt thereof.

### BRIEF DESCRIPTION OF THE PRESENT INVENTION

The present invention relates to an improved process for making ivabradine of formula (I) or an acid addition salt thereof.

The first aspect of the present invention is a process for making dehydroivabradine of formula (II) or an acid addition salt thereof 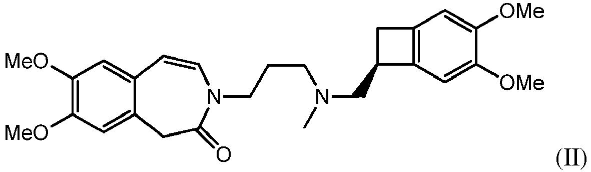 comprising reacting the compounds of formulae (III) and (IV) 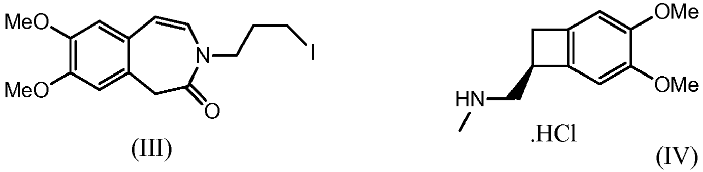 in the presence of a base in a biphasic solvent system, wherein the biphasic solvent system comprises a mixture of water and a hydrocarbon, preferably a mixture of water and toluene.

In an advantageous embodiment, dehydroivabradine of formula (II) is recovered from the reaction mixture as an aqueous solution of a water-soluble acid addition salt, preferably an aqueous solution of dehydroivabradine hydrochloride.

In a further advantageous embodiment, dehydroivabradine of formula (II) is recovered from the reaction mixture in an isolated, advantageously solid, form of dehydroivabradine hydrochloride.

In yet another embodiment, ivabradine of formula (I) or an acid addition salt thereof 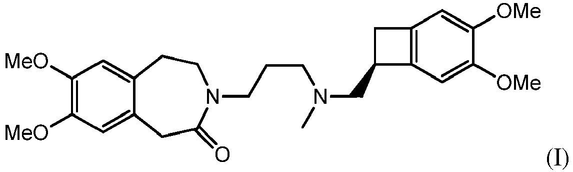 is prepared by a process comprising subjecting the aqueous solution of a water-soluble salt of dehydroivabradine of formula (II), preferably an aqueous solution of dehydroivabradine hydrochloride, to a hydrogenation reaction with gaseous hydrogen in the presence of a hydrogenation catalyst, advantageously at a hydrogen pressure not exceeding 1.5 bar (i.e. 0.15 MPa). Preferably, the hydrogenation catalyst is a palladium catalyst.

In a particular embodiment, ivabradine is recovered from the reaction mixture as a solution of ivabradine free base in a water-immiscible organic solvent, advantageously an ester solvent, more advantageously isopropyl acetate.

### DETAILED DESCRIPTION OF THE INVENTION

The present invention relates to an improved process for making ivabradine of formula (I) or an acid addition salt thereof. In particular, it relates to an improved process for making the key intermediate in said process, i.e. dehydroivabradine of formula (II) or an acid addition salt thereof, as well as to an improved process for converting said dehydroivabradine of formula (II) into ivabradine.

The process for making the compound of formula (II) according to the present invention is characterized in that the condensation reaction between compounds (III) and (IV) proceeds in a biphasic solvent system, advantageously in a toluene-water mixture. This process for making dehydroivabradine and subsequently ivabradine is superior over any of the prior art procedures in that it proceeds in a substantially shorter reaction time and the work-up of the reaction mixture does not require any specific treatment associated with isolation of the intermediate compound (II). In particular, the process effectively suppresses the presence of the "overalkylation impurity" of formula (V) (X = Cl or I) as well as of its demethylation - product of formula (VI) - which is presumably caused by the action of iodine ions present in the reaction mixture.

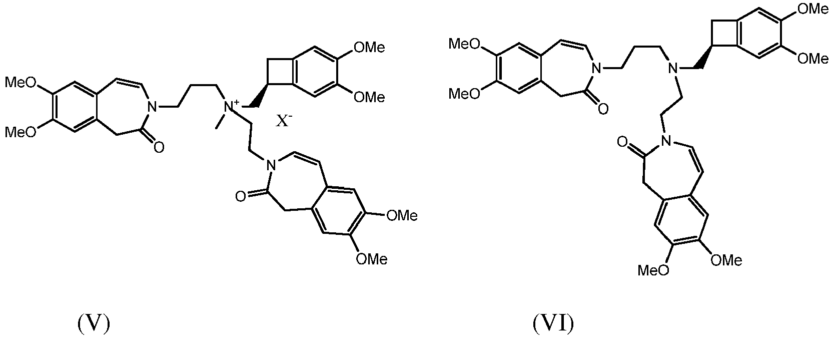

While the compound (V) is typically formed in 5-10% amount during the reaction between (III) and (IV) and is present in this amount in reaction mixture in prior art process, it is not soluble in the biphasic solvent system used in accordance with the present invention. It therefore does not react further to (VI) (which undesired reaction is common in the prior art process), and also can be easily separated from the reaction mixture by means of filtration.

In summary, the process of making compound (II) according to the present invention is more economical vis-a-vis any of the prior art processes.

The known process of catalytic hydrogenation of dehydroivabradine of formula (II) to ivabradine of formula (I) requires using an organic solvent such as methanol or acetic acid, which has to be evaporated after termination of the reaction. The procedure of the present invention disclosed in more detail below, uses water as the hydrogenation medium, thus omits the use of any organic solvent and, furthermore, allows without need of prior isolation direct conversion of the dehydroivabradine salt into ivabradine free base and then into ivabradine hydrochloride .

The hydrogenation in water proceeds in an acceptable rate even at hydrogen pressure not exceeding 1.5 bar, which allows replacing expensive pressure vessels, the use of which being required in the prior art procedures, by conventional reaction vessels. The hydrogenation product is isolated from the aqueous reaction medium by simple extraction.

In summary, also the process of making the compound (I) according to the present invention is more economical vis-a-vis any of the prior art processes.

The processes according to the present invention will now be described in more detail.

The starting materials are the compounds of formula (III) and (IV), respectively. Both are known compounds and may either be obtained commercially or produced by processes known per se in the art. For instance, the iodo-compound of formula (III) may be prepared by reacting the chloro-compound of formula (IIIa) with sodium or potassium iodide in a suitable solvent, which is advantageously an aliphatic ketone. While the prior art (i.e. Example 1 of EP 534859) teaches using acetone as the solvent, the present inventors found that methyl isobutyl ketone is superior over acetone as the reaction time is shortened from 24 hours to 90 minutes.

The amine hydrochloride compound of formula (IV) may be prepared by various procedures known in the art. The molecule is optically active. Within the context of the process of the present invention, it should be used as a material of high optical purity, i.e. having a content of the desired (S)-enantiomer higher than 98%, and preferably higher than 99%.

Without limitation, the hydrochloride moiety in the compound of formula (IV) may be replaced by any other acid moiety, or may be completely omitted, i.e. the compound (IV) may be used as a free base. Hence, the compound of formula (IV) to be used in accordance with the present invention may be the hydrochloride or any other suitable acid addition salt of the amine or the free base.

In the process of the present invention, dehydroivabradine of formula (II) is made by reacting the compound of formula (III) with the compound of formula (IV) in the presence of a base in a biphasic solvent system.

The "biphasic solvent system" as used herein means a liquid medium consisting of two visually clearly distinguishable liquid phases, i.e. being a mixture of two mutually immiscible liquids or liquid mixtures. One phase is an aqueous phase, which typically comprises water, the other phase is an organic phase, which typically comprises a hydrocarbon.

The "hydrocarbon" is not particularly limited in terms of carbon numbers and preferably is selected from the group consisting of aliphatic hydrocarbons of from 5 to 10 carbon atoms, cyclic hydrocarbons of from 5 to 8 carbon atoms, aromatic hydrocarbons of from 6-10 carbon atoms, and mixtures thereof. A preferred hydrocarbon is hexane, cyclohexane or toluene.

The "base" to be used in accordance with the process of the present invention is preferably an inorganic base and more preferably is a carbonate, e.g. potassium or sodium carbonate or potassium or sodium hydrogencarbonate. The molar amount of base is advantageously 2-2.5 molar equivalents, with respect to compound of formula (III).

The molar ratio between compounds (III) and (IV) is typically 1 : 1.

The iodo-compound of formula (III) may be charged as isolated material but it may also be prepared *in situ,* preferably in the same, biphasic solvent system in advance, e.g. by reaction of the chloro-compound of formula (IIIa) with a metal iodide, e.g. sodium iodide, optionally under catalysis of a quaternary ammonium iodide, e.g., tetrabutylammonium iodide.

The reaction temperature is advantageously from 50 to 100°C, preferably from 60 to 90°C.

Advantageously, the reaction proceeds in an inert atmosphere, e.g. in argon atmosphere.

The reaction time depends on the temperature and solvent system used. The reaction progress may be monitored by a suitable analytical method, e.g. by HPLC, and the reaction may be terminated when completed.

As the reaction proceeds in a biphasic solvent system, good stirring is of a certain importance. Phase transfer catalysts are, in general, not required, but their use is not excluded. A suitable catalyst is a quaternary ammonium salt. Suitable salts are well-known to the person skilled in the art.

It should be noted that side products of the reaction, such as sodium iodide or chloride, remain dissolved in the aqueous phase, while the product of the reaction remains dissolved in the organic phase. Thus, both components may easily be separated by separation of the respective aqueous and organic phases after completion of the reaction. Without wishing to be bound by any theory, the present inventors speculate that shifting the reaction equilibrium caused by permanent removal of the side-products from the organic phase effectively improves the reaction rate in comparison to the prior art processes proceeding in a single organic phase.

The dehydroivabradine of formula (II) is advantageously recovered from the reaction mixture as an aqueous solution of a water-soluble acid addition salt, preferably as an aqueous solution of dehydroivabradine hydrochloride. Thus, the reaction mixture is typically filtered after termination of the reaction and both phases are separated. The organic phase comprising the desired product is extracted with an aqueous acid solution, typically with an aqueous hydrochloric acid solution, whereby the dehydroivabradine moves into the aqueous phase, wherein it stays dissolved in its salt form.

The aqueous solution comprising the dehydroivabradine salt may be used in the next reaction step without any further treatment, particularly without any chromatographic purification.

In case of need or desire, dehydroivabradine or a salt thereof, e.g., dehydroivabradine hydrochloride, may be recovered in an isolated form from the reaction mixture. For such purpose, the aqueous solution comprising, typically, dehydroivabradine hydrochloride is neutralized with a suitable base and extracted with a water-immiscible organic solvent, e.g. an aliphatic ester such as ethyl acetate or isopropyl acetate. Dehydroivabradine free base then can be isolated from the organic solution by evaporation of the solvent. The solution of dehydroivabradine free base in the organic solvent may also serve for making dehydroivabradine hydrochloride. According to present knowledge, the hydrochloride salt cannot crystallize from the solution of dehydroivabradine free base in the above organic solvent by simple adding hydrochloric acid, as it easily separates out as an oil. Instead, it was found that the solid, particularly crystalline, dehydroivabradine hydrochloride can be obtained by slowly adding the above solution of dehydroivabradine free base to an alcoholic solution of HCl at an elevated temperature, followed by cooling of the obtained reaction mixture.

The aqueous solution of dehydroivabradine salt, preferably dehydroivabradine hydrochloride, and in particular the aqueous solution obtained by the process disclosed above, can be used as the starting material in the process for making ivabradine according to the present invention. No conversion into dehydroivabradine free base, which is obligatory in the processes of the prior art, is necessary. Typically, no organic co-solvent is present in the aqueous solution.

The process will be illustrated starting from dehydroivabradine hydrochloride, but the invention is not limited to the use of this specific acid addition salt.

Thus, according to the present invention, an aqueous solution of dehydroivabradine salt, such as dehydroivabradine hydrochloride, which typically has a concentration of from 5 to 15 wt%, preferably about 10 wt%, and which was preferably prepared by the above process, is subjected to a hydrogenation reaction with gaseous hydrogen in the presence of a hydrogenation catalyst, which preferably is a palladium catalyst. Suitable palladium catalysts are, e.g., 5-10% palladium on carbon or 5-10% palladium hydroxide on carbon. The reaction proceeds advantageously under a hydrogen pressure not exceeding 1.5 bar, at ambient or slightly higher than ambient temperature, advantageously at a temperature of from 25-40°C.

The course of the reaction may be monitored by a suitable analytical method, e.g., by HPLC. Typically, the reaction time is about 5 hours.

After termination of the reaction, the catalyst is removed by filtration. The reaction mixture comprises an aqueous solution of ivabradine salt, typically ivabradine hydrochloride.

Ivabradine may be isolated from this reaction mixture in various solid state forms, either as a free base or as an acid addition salt. Conversion into ivabradine free base may also be done for purpose of purification.

Thus, for instance, the above aqueous solution of ivabradine hydrochloride is combined with a suitable water-immiscible organic solvent, for instance with a C3-C8 aliphatic ester such as ethyl acetate or isopropyl acetate, a C4-C8 aliphatic or cyclic ether such as diethyl ether or tetrahydrofuran, or with a C5-C10 hydrocarbon such as toluene or hexane, and the biphasic solvent mixture is neutralized with a suitable base, e.g. an aqueous sodium hydroxide solution. The organic layer comprising ivabradine free base is then separated and ivabradine free base is isolated, e.g., by evaporation of the solvent.

With respect to acid addition salts of ivabradine obtainable by isolation from the above reaction mixture or from the above solution of ivabradine free base, the preferred salt is ivabradine hydrochloride, which can be obtained in various polymorphic forms.

It is preferred that the formed crystals have an average particle size of less than 1,000 microns, preferably less than 250 microns, and more preferably less than 100 microns. The product of small particle size is, in general, easier to process into pharmaceutical compositions than when a product having a large particle size is used. If such particle size is not obtainable by the process itself, conventional operations like sieving or milling can be performed to achieve a desired particle size or particle size distribution.

The ivabradine hydrochloride obtained by the process of the present invention can be formulated into various pharmaceutical compositions with one or more pharmaceutically acceptable excipients. The pharmaceutical composition can be in a unit dosage form, such as a solid oral dosage form (i.e. a tablet or capsule), or a bulk precursor thereof, such as a preblended mixture ready for further blending/addition of ingredients or a blend ready for tabletting or filling into capsules. Pharmaceutically acceptable excipients may include, without limitation, carriers, diluents, fillers, binders, lubricants, disintegrants, glidants, colorants, pigments, taste masking agents, sweeteners, flavorants, plasticizers, etc. The proper excipient(s) are selected based in part on the desired dosage form, the intended mode of administration, the intended release rate, and manufacturing reliability.

The ivabradine hydrochloride preferably is formulated into pharmaceutical compositions for oral administration. The compositions for oral administration may be solid or liquid, such as in the form of an oral solution or suspension, oral capsule, or an oral tablet, and may exhibit immediate release or modified and/or extended release of the active substance from the composition.

The pharmaceutical dosage forms formulated from the compositions may comprise a unit dose of ivabradine, e.g., a therapeutically effective amount of ivabradine hydrochloride for a single dose administration. The amount of ivabradine hydrochloride, expressed as ivabradine free base, in the daily unit dose may range from 0.1 to 20 mg, typically from 1 to 10 mg.

The ivabradine hydrochloride prepared by the process of the present invention or pharmaceutical compositions comprising it can be used in medicine, for instance for the treatment of angina pectoris, in particular symptomatic treatment of chronic stable angina pectoris. It may be used in monotherapy or in combination therapy.

The invention will be further illustrated by way of the following non-limiting examples.

### EXAMPLES

### Preparation of the compound of formula (III)

A mixture of the chloride of formula (IIIa) (50.00 g; 169 mmol) and sodium iodide (32.75 g; 218 mmol) in methyl isobutyl ketone (375 ml) was heated to 117 - 118°C with stirring under an argon atmosphere. Reaction progress was monitored by HPLC. After completion, the reaction mixture was concentrated *in vacuo* and the residue was diluted with dichloromethane (375 ml) and water (190 ml). The organic layer was separated, washed with water (190 ml) and concentrated *in vacuo.* The residue was treated with methyl *t*-butyl ether (190 ml) and the resulting suspension was cooled to 0°C. After 1 hour of stirring at 0°C, the suspension was filtered, washed with methyl *t*-butyl ether (40 ml) and dried for two hour at 23°C/100 mbar to afford 60.10 g of yellowish solid (92% yield).

### Example 1

### Preparation of aqueous dehydroivabradine hydrochloride solution

A mixture of the iodo-compound of formula (III) (50.00 g; 129 mmol), amine hydrochloride of formula (IV) (31.50 g; 129 mmol), potassium carbonate (37.70 g; 273 mmol), toluene (80 ml) and water (40 ml) was heated to 70°C with stirring under an argon atmosphere. Reaction progress was monitored by HPLC. Upon completion, the reaction mixture was cooled to 23°C, diluted with toluene (105 ml), and filtered through a pad of celite. The filter cake was washed with additional toluene (60 ml). To the filtrate, water (50 ml) was added, and the mixture was shaken. The organic layer was separated and washed with additional water (80 ml). The separated organic layer was then extracted with 9% aqueous hydrochloric acid (507 g) in three portions. Yield of dehydroivabradine hydrochloride in combined acidic extracts was calculated based on HPLC assay using an external standard. The product was obtained in 80% yield as an aqueous solution which was used directly in the next step.

### Example 2

### Preparation of aqueous ivabradine free base solution

To a 10.2wt% solution of dehydroivabradine hydrochloride in water (318.00 g; 64 mmol), 20% palladium hydroxide on carbon (50% wet, 3.07 g; 2 mmol) was added, and the reaction mixture was flushed with argon followed by hydrogen. Then, the reaction mixture was stirred at 30°C under a balloon of hydrogen for 5 hours. Then, the reaction mixture was filtered through a pad of celite, and the filter cake was washed with water (10 ml) and the filtrate was subsequently combined with isopropyl acetate (288 ml). The mixture was cooled to 15°C and 30% aqueous sodium hydroxide (10.41 g) was added slowly at 15°C. The mixture was stirred for an additional 30 minutes, and then the organic layer was separated and the aqueous layer was extracted with isopropyl acetate (144 ml). The combined organic extracts were heated to 71 - 81°C, and the solvent was partially distilled off to obtain a 10wt% solution of ivabradine free base in isopropyl acetate, which was used directly in the next step. Yield of the product was calculated based on HPLC assay using an external standard. The product was obtained in 91% yield.

### Example 3

### Ivabradine hydrochloride Delta-d polymorph

A 25 ml two neck round bottom flask equipped with a magnetic stir bar, a condenser, and a thermocouple was charged with isopropyl acetate solution of ivabradine free base (4.59 g of 21.80% solution, i.e. 1 g of ivabradine free base; 2.135 mmol) followed with isopropyl acetate (4 ml) and ethanol (4 ml) and the mixture was heated to 50°C. The reaction mixture was cooled to 0°C and additional isopropyl acetate (4 ml) was added at 0°C and the reaction mixture was then seeded. 35% hydrochloric acid (0.208 ml, 2.349 mmol) was added at 0°C whereupon precipitation was observed. The suspension was stirred at 0°C for additional 1 hour and then filtered and dried in the drier (23°C, 130 mbar, N₂ bleed, 20 hours) to give 880 mg of Delta-d polymorph of ivabradine hydrochloride in the form of white crystalline solid (81% of theory).

### Example 4

### Preparation of dehydroivabradine hydrochloride

A1 L round-bottomed flask equipped with a mechanical stirrer and a condenser was charged with ethanol (40 ml) and concentrated hydrochloric acid (4.03 ml, 47.3 mmol). The mixture was heated to 60°C (65°C in oil bath) under an argon atmosphere. An isopropyl acetate solution of dehydroivabradine (200 g of 10% solution, 43.0 mmol) was added via an addition funnel over 70 minutes. The reaction mixture was cooled to 23°C and the resulting emulsion was seeded with dehydroivabradine.HCl. The mixture was stirred for 69 hours. The precipitated solid was filtered using a Buchner funnel, and the filter cake was washed with isopropyl acetate (40.0 ml). The filter cake was dried (24°C, 140 mbar, N₂, 2 hr) to give 15.47 g of a yellowish solid (71.5% yield).

DSC (20-200°C, 10°C/min, standard cell): single melting endotherm at 150.0°C

## Claims

1. A process for making dehydroivabradine of formula (II) or an acid addition salt thereof 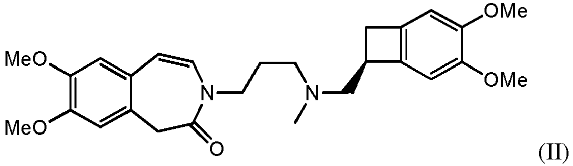 comprising reacting the compounds of formulae (III) and (IV) 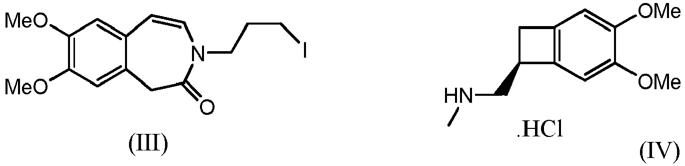 in the presence of a base in a biphasic solvent system, wherein the biphasic solvent system comprises a mixture of water and a hydrocarbon, preferably a mixture of water and toluene.

2. The process according to claim 1, wherein the hydrocarbon is selected from at least one aliphatic hydrocarbon of from 5-10 carbon atoms, at least one cyclic hydrocarbon of from 5-8 carbon atoms, at least one aromatic hydrocarbon of from 6-10 carbon atoms, and mixtures thereof, preferably the hydrocarbon is toluene.

3. The process according to claim 1 or 2, wherein the base is an inorganic base, preferably a carbonate.

4. The process according to any one of claims 1-3, wherein the reaction temperature is from 50 to 100°C, preferably from 60 to 90°C.

5. The process according to any one of claims 1-4, wherein the dehydroivabradine of formula (II) is recovered from the reaction mixture as an aqueous solution of a water-soluble acid addition salt thereof, preferably an aqueous solution of dehydroivabradine hydrochloride.

6. The process according to any one of claims 1-5, wherein dehydroivabradine hydrochloride is recovered from the reaction mixture in an isolated, advantageously solid, form.

7. A process according to claim 5 further comprising the step of making ivabradine of formula (I) or an acid addition salt thereof 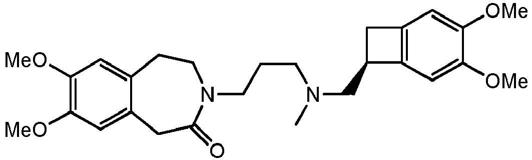 comprising subjecting the aqueous solution of a water-soluble salt of dehydroivabradine of formula (II), preferably an aqueous solution of dehydroivabradine hydrochloride, to a hydrogenation reaction with gaseous hydrogen in the presence of a hydrogenation catalyst.

8. The process according to claim 7, wherein the aqueous solution of a water-soluble dehydroivabradine salt is obtained by the process according to any one of claims 1-5.

9. The process according to claim 7 or 8, wherein the hydrogenation catalyst is a palladium catalyst.

10. The process according to any one of claims 7-9, wherein the hydrogen pressure does not exceed 1.5 bar.

11. The process according to any one of claims 7-10, wherein ivabradine is recovered from the reaction mixture as a solution of ivabradine free base in a water-immiscible organic solvent.

12. The process according to claim 11, wherein the solution of ivabradine free base is contacted with HCl upon formation of crystalline ivabradine hydrochloride.

## Patentansprüche

1. Verfahren zum Herstellen von Dehydroivabradin der Formel (II) oder eines Säureadditionssalzes davon 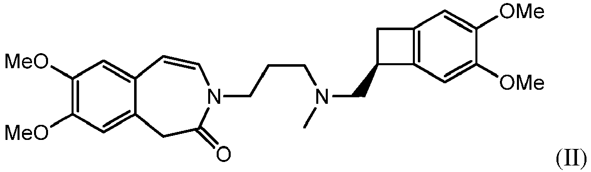 umfassend Umsetzen der Verbindungen der Formeln (III) und (IV) 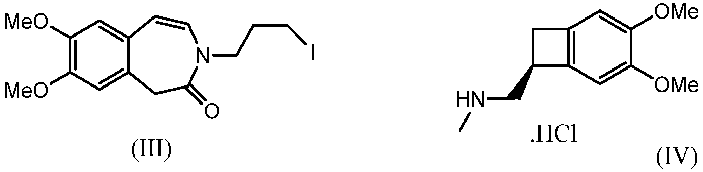 in der Gegenwart einer Base in einem biphasischen Lösungsmittelsystem, wobei das biphasische Lösungsmittelsystem ein Gemisch aus Wasser und einem Kohlenwasserstoff umfasst, bevorzugt ein Gemisch aus Wasser und Toluol.

2. Verfahren nach Anspruch 1, wobei der Kohlenwasserstoff aus mindestens einem aliphatischen Kohlenwasserstoff von 5-10 Kohlenstoffatomen, mindestens einem cyclischen Kohlenwasserstoff von 5-8 Kohlenstoffatomen, mindestens einem aromatischen Kohlenwasserstoff von 6-10 Kohlenstoffatomen, und Gemischen davon ausgewählt ist, bevorzugt ist der Kohlenwasserstoff Toluol.

3. Verfahren nach Anspruch 1 oder 2, wobei die Base eine anorganische Base ist, bevorzugt ein Carbonat.

4. Verfahren nach einem beliebigen der Ansprüche 1-3, wobei die Reaktionstemperatur von 50 bis 100°C beträgt, bevorzugt von 60 bis 90°C.

5. Verfahren nach einem beliebigen der Ansprüche 1-4, wobei das Dehydroivabradin der Formel (II) aus dem Reaktionsgemisch als eine wässrige Lösung eines wasserlöslichen Säureadditionssalzes davon gewonnen wird, bevorzugt eine wässrige Lösung von Dehydroivabradin-Hydrochlorid.

6. Verfahren nach einem beliebigen der Ansprüche 1-5, wobei Dehydroivabradin-Hydrochlorid aus dem Reaktionsgemisch in einer isolierten, vorteilhafterweise festen Form gewonnen wird.

7. Verfahren nach Anspruch 5, das des Weiteren den Schritt des Herstellens von Ivabradin der Formel (I) oder eines Säureadditionssalzes davon umfasst 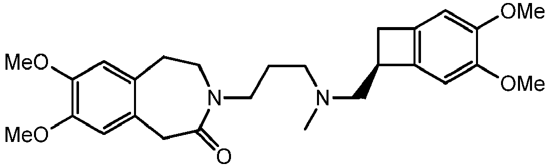 der Unterziehen der wässrigen Lösung eines wasserlöslichen Salzes von Dehydroivabradin der Formel (II), bevorzugt einer wässrigen Lösung von Dehydroivabradin-Hydrochlorid, einer Hydrierungsreaktion mit gasförmigem Wasserstoff in der Gegenwart eines Hydrierkatalysators umfasst.

8. Verfahren nach Anspruch 7, wobei die wässrige Lösung eines wasserlöslichen Dehydroivabradinsalzes durch das Verfahren gemäß einem beliebigen der Ansprüche 1-5 erhalten wird.

9. Verfahren nach Anspruch 7 oder 8, wobei der Hydrierkatalysator ein Palladiumkatalysator ist.

10. Verfahren nach einem beliebigen der Ansprüche 7-9, wobei der Wasserstoffdruck nicht 1,5 bar überschreitet.

11. Verfahren nach einem beliebigen der Ansprüche 7-10, wobei Ivabradin aus dem Reaktionsgemisch als eine Lösung von freier Base von Ivabradin in einem in Wasser unmischbaren organischen Lösungsmittel gewonnen wird.

12. Verfahren nach Anspruch 11, wobei die Lösung von freier Base von Ivabradin mit HCl bei Bildung von kristallinem Dehydroivabradin-Hydrochlorid in Kontakt gebracht wird.

## Revendications

1. Un procédé de préparation de la déhydroivabradine de formule (II) ou d'un sel d'addition d'acide de celle-ci 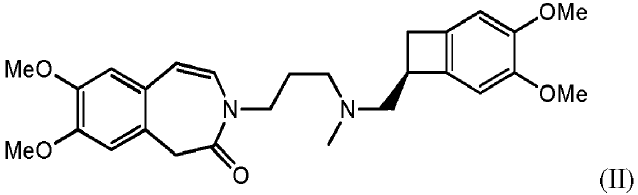 comprenant la réaction des composés de formules (III) et (IV) 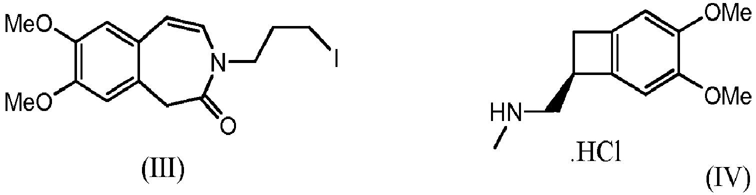 en présence d'une base dans un système de solvant biphasique dans lequel le système de solvant biphasique comprend un mélange d'eau et d'un hydrocarbure, de préférence un mélange d'eau et de toluène.

2. Le procédé selon la revendication 1, dans lequel l'hydrocarbure est choisi parmi au moins un hydrocarbure aliphatique de 5 à 10 atomes de carbone, au moins un hydrocarbure cyclique de 5 à 8 atomes de carbone, au moins un hydrocarbure aromatique de 6 à 10 atomes de carbone, et leurs mélanges, de préférence l'hydrocarbure est le toluène.

3. Le procédé selon la revendication 1 ou la revendication 2, dans lequel la base est une base inorganique, de préférence un carbonate.

4. Le procédé selon l'une quelconque des revendications 1 à 3, dans lequel la température de réaction est de 50 à 100°C, de préférence de 60 à 90°C.

5. Le procédé selon l'une quelconque des revendications 1 à 4, dans lequel la déhydroivabradine de formule (II) est récupérée du mélange réactionnel sous la forme d'une solution aqueuse d'un de ses sels d'addition d'acide hydrosoluble, de préférence une solution aqueuse de chlorhydrate de déhydroivabradine.

6. Le procédé selon l'une quelconque des revendications 1 à 5, dans lequel le chlorhydrate de déhydroivabradine est récupéré du mélange réactionnel sous une forme isolée, avantageusement solide.

7. Un procédé selon la revendication 5, comprenant en outre l'étape de fabrication de l'ivabradine de formule (I) ou d'un sel d'addition d'acide de celle-ci: 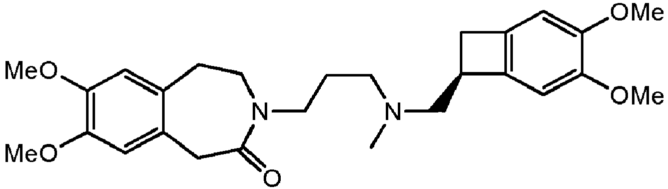 comprenant la soumission de la solution aqueuse d'un sel hydrosoluble de déhydroivabradine de formule (II), de préférence une solution aqueuse de chlorhydrate de déhydroivabradine, à une réaction d'hydrogénation avec de l'hydrogène gazeux en présence d'un catalyseur d'hydrogénation.

8. Le procédé selon la revendication 7, dans lequel la solution aqueuse d'un sel de déhydroivabradine hydrosoluble est obtenue par le procédé selon l'une quelconque des revendications 1-5.

9. Le procédé selon la revendication 7 ou 8, dans lequel le catalyseur d'hydrogénation est un catalyseur au palladium.

10. Le procédé selon l'une quelconque des revendications 7 à 9, dans lequel la pression d'hydrogène ne dépasse pas 1,5 bar.

11. Le procédé selon l'une quelconque des revendications 7 à 10, dans lequel l'ivabradine est récupérée à partir du mélange réactionnel sous la forme d'une solution de base libre d'ivabradine dans un solvant organique non miscible à l'eau.

12. Le procédé selon la revendication 11, dans lequel la solution de base libre d'ivabradine est mise en contact avec du HCl lors de la formation du chlorhydrate d'ivabradine cristallin.
